# EUROPEAN PATENT SPECIFICATION

(11) **EP 3 597 230 B1**
(45) Date of publication and mention of the grant of the patent: **24.04.2024**
(21) Application number: 18909859.3
(22) Date of filing: 13.07.2018
(51) Int. Cl.: A61M 1/06, A61M 1/00, A61M 39/24, A61F 2/52

(54) **SMART WEARABLE BREAST PUMP**
INTELLIGENTE, AM KÖRPER TRAGBARE BRUSTPUMPE
TIRE-LAIT PORTABLE INTELLIGENT

(30) Priority: 14.03.2018 KR 20180029649; 14.03.2018 KR 20180029650; 09.05.2018 KR 20180053080; 09.05.2018 KR 20180053114; 09.07.2018 KR 20180079224
(43) Date of publication of application: 22.01.2020
(73) Proprietor: Cimilre Co., Ltd., Cheonan-si, Chungcheongnam-do 31234 (KR)
(72) Inventor: KIM, Sang Ha, Seongnam-si, Gyeonggi-do 13441 (KR)
(74) Representative: González López-Menchero, Álvaro Luis
(86) International application number: PCT/KR2018/007964
(87) International publication number: WO 2019/177208

(56) References cited:
- WO-A1-2017/139437
- KR-A- 20100 017 650
- KR-A- 20170 044 650
- KR-B1- 100 383 401
- KR-B1- 101 622 768
- KR-B1- 101 660 362
- US-A- 6 042 537
- US-A1- 2002 188 231
- US-A1- 2002 193 731
- US-A1- 2008 208 116

## Description

### BACKGROUND OF THE INVENTION

### Field of the Invention

The present invention relates to a smart wearable breast pumping machine.

### Background of the Related Art

Recently, a breast pump is widely used when it is hard to directly feed a mother's breast milk to a baby or so as to separately store the breast milk. Generally, the breast pump includes a feeding cup for surrounding the breast, a feeding bottle for collecting the breast milk, and a driving device for generating a driving force to collect the breast milk, and the breast pump is classified into a manual breast pump and an automatic breast pump.

The driving device of the manual breast pump has a form of a lever or air bag-shaped mechanism, and as the mechanism is repeatedly grasped and not grasped by a user's hand, a vacuum pressure is generated. As a result, the breast milk is expressed by means of the generated vacuum pressure and is thus stored in the feeding bottle. So as to allow the driving device of the manual type breast pump to operate, however, the driving device has to be repeatedly grasped and not grasped by the user's hand, thereby causing many inconveniences of use. Further, the breast pumping function of the driving device is not really excellent, thereby making it hard to pump the breast milk.

On the other hand, the driving device of the automatic breast pump includes an electric motor and a pump, and one of conventional driving devices of the automatic breast pump is disclosed in Korean Utility Model Registration No.20-0219781 entitled "breast milking machine". The conventional breast milking machine includes a milking part having an attachment tube disposed on one side thereof to cover a mother's feeding portion (breast), a coupling part disposed on a lower side thereof in such a manner as to be fastened to a feeding bottle, and a backflow prevention valve fastened to a breast milk discharge hole formed on a bottom of an internal space divided into both spaces by means of a partition to prevent the breast milk stored in the feeding bottle from flowing back to the outside of the feeding bottle, vacuum pumping means connected as a tube to a sucking and discharging hole formed on one side communicating with the internal space of the milking part in such a manner as to be separated to a given length and adapted to perform and release vacuum pumping for the air filled in the internal space of the milking part with a given repeated cycle so as to periodically express the breast milk, and piston driving means for performing the vacuum pumping of the vacuum pumping means.

It is also known in the state of the art the following documents:
- WO2017139437 which discloses Systems and methods for pumping milk from a breast, wherein the milk is expressed from the breast under suction and milk is expulsed from the pumping mechanism to a collection container under positive pressure.
- US2002193731 which discloses a portable, hands-free and user friendly breast pump for facilitating breastfeeding, incorporates a dome-shaped housing having a servomotor mechanism; a hat-shaped flange having a chamber portion, a brim portion and an outlet, the chamber portion being formed so as to be placed over a nipple on a breast so as to define a chamber between the flange and the breast.
- US2008208116 which discloses a compact and hands-free human breast milk collection device that fits into a mother's existing nursing or standard brassiere

However, the breast pumps in the conventional practices do not provide various breast pumping methods according to the user's needs and also do not give more accurate and useful breast pumping information to the user.

Besides, the breast pumps in the conventional practices provide only the breast pumping function, thereby failing to have customized functions, such as nipple correction, breast enhancement, and breast massage, which are needed by the user.

### SUMMARY OF THE INVENTION

Accordingly, the present invention has been made in view of the above-mentioned problems occurring in the related art, and it is an object of the present invention to provide a smart wearable breast pumping machine that is capable of having an integral type structure in which a breast pump is built and also having a separate type structure in which a breast pump is not built therein and is provided as a separate type breast pumping device, thereby making breast pumping environments corresponding to a variety of needs of a user.

It is another object of the present invention to provide a smart wearable breast pumping machine that is capable of receiving and controlling information thereon through an application installed on a user terminal, thereby providing more accurate and useful breast pumping information for a user.

It is yet another object of the present invention to provide a nipple correction and breast enhancement machine that is capable of allowing a user to select appropriate correction according to the user's need, thereby providing various customized correction.

The invention is defined by the features of independent claim 1.

To accomplish the above-mentioned objects, according to one aspect of the present invention, there is provided a smart wearable breast pumping machine including: a suction part attached to a user's breast; a connector connected to the suction part to introduce breast milk pumped by means of a breast pumping pressure and coupled to a breast milk container; and a breast pumping part coupled to the suction part to accommodate the connector and having a breast pump adapted to apply the breast pumping pressure to the suction part and a controller adapted to control operations of the breast pump by means of control commands.

According to the present invention, desirably, the breast pumping part is separated from the suction part and the connector to constitute a separate breast pumping device.

According to the present invention, desirably, the suction part and the connector are separated from the breast pumping part to constitute a separate breast milk collector, and the connector receiving the breast pumping pressure from the breast pumping part through an air hose transfers the breast pumping pressure to the suction part to allow the pumped breast milk to be collected to the breast milk collector.

According to the present invention, the connector includes: a backflow prevention part formed integrally with a coupled portion to the air hose; and a backflow prevention elastic member (160),(261)inserted into the backflow prevention part.

According to the present invention, the breast pumping part further includes a communication module adapted to transmit breast pumping information of the breast milk and operation information of the breast pump to a user terminal and to receive the control commands from the user terminal.

To accomplish the above-mentioned objects, according to another aspect of the present invention, there is provided a smart wearable breast pumping machine including: a suction part attached to a user's breast; a breast pump for applying a breast pumping pressure to the suction part; a connector connected to the suction part to introduce breast milk pumped by means of the breast pumping pressure; a controller for controlling operations of the breast pump by means of control commands; and a cover part coupled to the suction part to accommodate the breast pump, the connector, and the controller therein.

According to the present invention, the smart wearable breast pumping machine further includes a backflow prevention part disposed on the connector to prevent foreign matters from flowing back by means of the breast pumping pressure applied to the connector from the breast pump.

According to the present invention, the backflow prevention part includes: a case extended from the connector in such a manner as to be formed unitarily with the connector; a cap extended from the cover part in such a manner as to be formed unitarily with the cover part, coupled to the case, and coupled to an air hose connected to the breast pump; and an elastic member disposed in an interior formed by coupling the case and the cap to each other in such a manner as to be elastically deformed by means of the breast pumping pressure applied from the breast pump through the air hose.

According to the present invention, desirably, the cover part is formed unitarily with a breast pump outer appearance portion for forming an outer appearance of the breast pump, a controller housing for accommodating the controller, and a battery housing for accommodating a battery applying power to the breast pump, on the inside coupled to the suction part.

According to the present invention, desirably, the smart wearable breast pumping machine further includes a stopper or breast milk container coupled to the connector.

To accomplish the above-mentioned objects, according to yet another aspect not falling under the claimed invention, there is provided a nipple correction and breast enhancement machine including: a suction part attached to a user's breast; a pump for generating a sucking pressure; a pressure application part connected to the suction part to apply the sucking pressure generated from the pump to the suction part; and a controller for controlling operations of the pump through control commands.

The nipple correction and breast enhancement machine further includes: a coupling part coupled to the suction part and having an integral type structure in which a pump support for supporting the pump, the pressure application part, and a battery housing for accommodating the battery are formed integrally with each other; and a cover part coupled to the coupling part to accommodate the pump, the pressure application part, an air hose for connecting the pump and the pressure application part, the battery housing, and the controller therein.

The pressure application part is cylindrical in such a manner as to be open at the side toward the suction part and has a pump connector disposed on the other surface to the open side in such a manner as to be connected to the pump through the air hose.

The nipple correction and breast enhancement machine further includes a breast molding part communicating with the suction part in such a manner as to come into contact with the user's breast to mold the user's breast by means of the sucking pressure applied from the pressure application part, the pressure application part further including: a pump connector connected to the pump through the air hose; and a suction hole formed on the other end in which the pump connector is disposed to apply the sucking pressure generated from the pump to the breast molding part.

### BRIEF DESCRIPTION OF THE DRAWINGS

The above and other objects, features and advantages of the present invention will be apparent from the following detailed description of the embodiments of the invention in conjunction with the accompanying drawings, in which:
FIGS.1 and 2 are perspective views showing an outer appearance of a smart wearable breast pumping machine according to a first embodiment of the present invention;
FIG.3 is a perspective view showing a bottom portion of the smart wearable breast pumping machine according to the first embodiment of the present invention;
FIG.4 is a perspective view showing an interior of the smart wearable breast pumping machine according to the first embodiment of the present invention;
FIG.5 is a front view showing the interior of the smart wearable breast pumping machine according to the first embodiment of the present invention;
FIG.6 is a sectional view showing the smart wearable breast pumping machine according to the first embodiment of the present invention;
FIG.7 is an exploded perspective view showing the smart wearable breast pumping machine according to the first embodiment of the present invention;
FIG.8 is a perspective view showing a coupled state between the smart wearable breast pumping machine according to the first embodiment of the present invention and a breast milk container in case of the smart wearable breast pumping machine with an integral type;
FIG.9 is an exploded perspective view showing the smart wearable breast pumping machine according to the first embodiment of the present invention in case of the smart wearable breast pumping machine with a separate type;
FIG.10 is a perspective view showing a method for using the separate type the smart wearable breast pumping machine according to the first embodiment of the present invention;
FIG.11 is a perspective view showing a communication method between the integral type smart wearable breast pumping machine according to the first embodiment of the present invention and a user terminal;
FIG.12 is a perspective view showing a communication method between the separate type smart wearable breast pumping machine according to the first embodiment of the present invention and a user terminal;
FIGS.13 and 14 are perspective views showing an outer appearance of a smart wearable breast pumping machine according to a second embodiment of the present invention;
FIG.15 is a perspective view showing a bottom portion of the smart wearable breast pumping machine according to the second embodiment of the present invention;
FIG.16 is a perspective view showing an interior of the smart wearable breast pumping machine according to the second embodiment of the present invention;
FIG.17 is a front view showing the interior of the smart wearable breast pumping machine according to the second embodiment of the present invention;
FIG.18 is a sectional view showing the smart wearable breast pumping machine according to the second embodiment of the present invention;
FIG.19 is a perspective view showing a smart wearable breast pumping machine according to a third embodiment of the present invention;
FIG.20 is a sectional view showing the smart wearable breast pumping machine according to the third embodiment of the present invention;
FIG.21 is a perspective view showing a smart wearable breast pumping machine according to a fourth embodiment of the present invention;
FIG.22 is a sectional view showing the smart wearable breast pumping machine according to the fourth embodiment of the present invention;

### DETAILED DESCRIPTION OF THE PREFERRED EMBODIMENTS

Now, an explanation on a smart wearable breast pumping machine according to the present invention will be given with reference to the attached drawings. If it is determined that the detailed explanation on the well-known technology related to the present invention makes the scope of the present invention not clear, the explanation will be avoided for the brevity of the description. In the description, the sizes of the components shown in the drawing may be magnified for the clarity and convenience of the description, and therefore, they do not mean the sizes really applied.

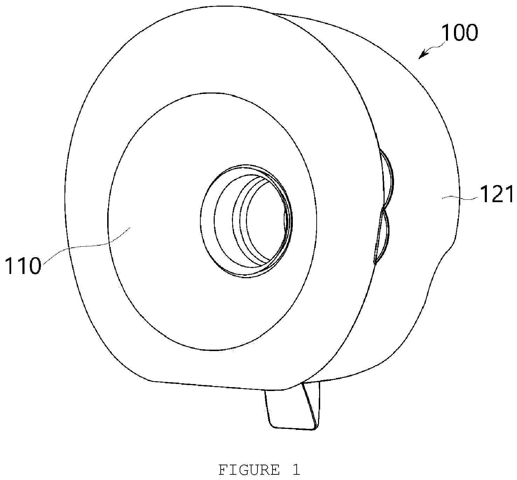

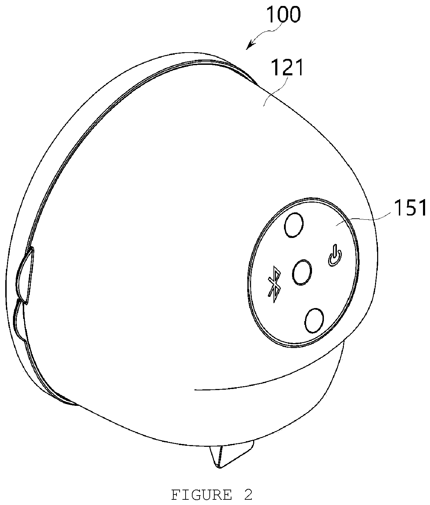

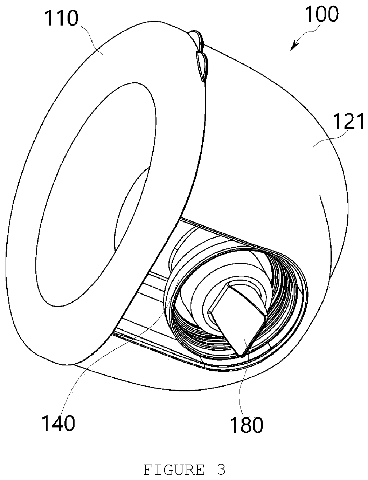

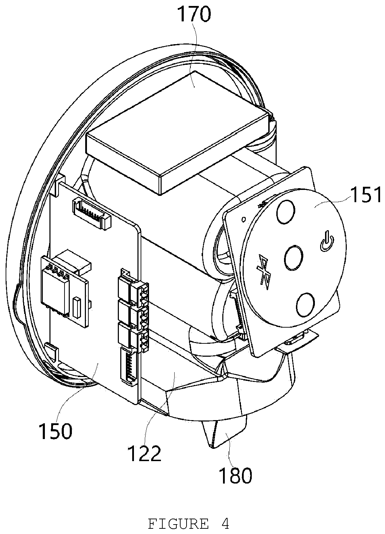

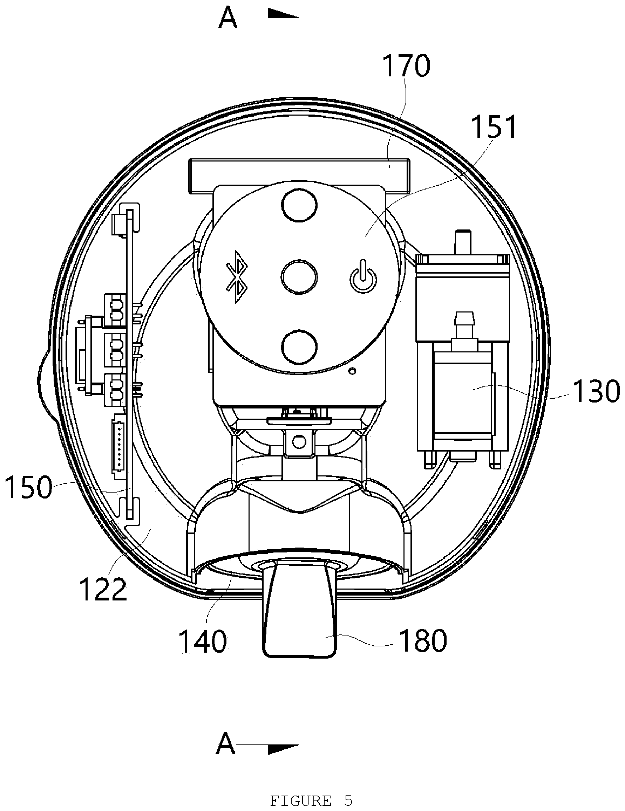

FIGS.1 and 2 are perspective views showing an outer appearance of a smart wearable breast pumping machine according to a first embodiment of the present invention, FIG.3 is a perspective view showing a bottom portion of the smart wearable breast pumping machine according to the first embodiment of the present invention, FIG.4 is a perspective view showing an interior of the smart wearable breast pumping machine according to the first embodiment of the present invention, and FIG.5 is a front view showing the interior of the smart wearable breast pumping machine according to the first embodiment of the present invention.

As shown in FIGS.1 to 5, a smart wearable breast pumping machine 100 according to a first embodiment of the present invention includes a suction part 110, a cover part 121, a breast pumping part 122, a breast pump 130, a connector 140, a controller 150, and a backflow prevention elastic member 160.

As shown in FIGS.1 and 2, the suction part 110 and the cover part 121 are coupled to each other, and a button type command input part 151 is disposed on the cover part 121 to control operation, stop, and strength adjustment for the breast pumping machine 100.

The suction part 110 comes into close contact with a user's breast and is thus suckedly attached thereto. Further, the suction part 110 receives a breast pumping pressure from the breast pump 130 and thus pumps breast milk.

As shown in FIG.3, the connector 140 has one side connected to the suction part 110 to introduce the breast milk pumped by means of the breast pumping pressure therein and the other side coupled to a breast milk container such as a feeding bottle, a breast milk storage pack, and the like.

Accordingly, the breast pumping pressure received from the breast pump 130 is applied to the suction part 110 attached to the user's breast, thereby allowing the breast milk to be pumped, and the pumped breast milk is introduced and stored in the breast milk container through the connector 140 and a silicone head 180, sequentially.

As shown in FIGS. 4 and 5, the breast pumping part 122 includes the breast pump 130 for applying the breast pumping pressure to the suction part 110 and the controller 150 for controlling operations of the breast pump 130 according to control commands of the command input part 151.

Further, the controller 150 controls the breast pump 130 through the control commands to allow the operation, stop, and strength adjustment for the breast pumping machine 100 to be controlled, and the command input part 151 for inputting the control commands of the breast pumping machine 100 is exposed to the outside through the cover part 120.

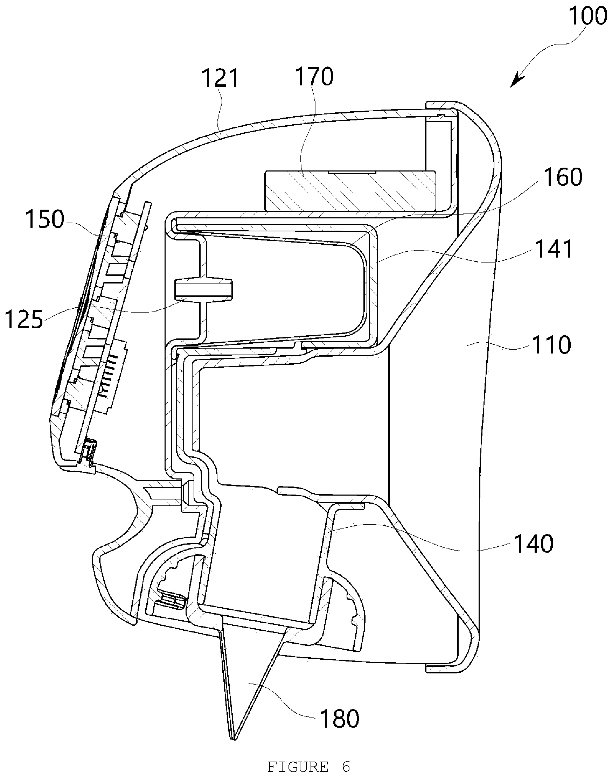

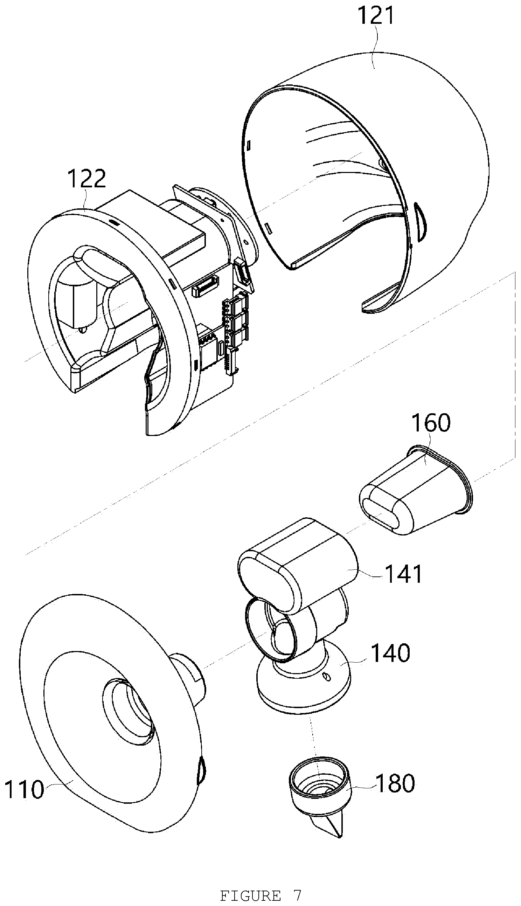

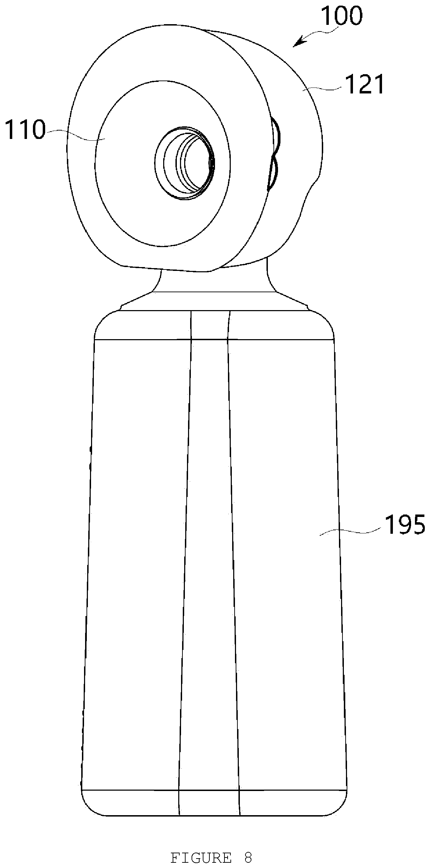

FIG.6 is a sectional view showing the smart wearable breast pumping machine according to the first embodiment of the present invention, which is taken along the line A-A' of FIG.5, FIG.7 is an exploded perspective view showing the smart wearable breast pumping machine according to the first embodiment of the present invention, and FIG.8 is a perspective view showing a coupled state between the smart wearable breast pumping machine according to the first embodiment of the present invention and a breast milk container in case of the smart wearable breast pumping machine with an integral type.

As shown in FIGS.6 and 7, the connector 140 is connected to the suction part 110 attached to the user's breast to introduce the breast milk pumped by means of the breast pumping pressure received from the breast pumping part 122 therein, and the connector 140 is also coupled to the breast pumping part 122.

The breast pumping part 122 generates the breast pumping pressure and applies the generated breast pumping pressure to the suction part 110 through the connector 140, so that the breast milk can be pumped.

Moreover, the connector 140 is formed integrally with a backflow prevention part 141. The backflow prevention part 141 serves to block the introduction of foreign matters, and the backflow prevention elastic member 160 made of a silicone material is insertedly disposed in the backflow prevention part 141. Under the above-mentioned configuration, the breast pumping part 122 is coupled to the cover part 121, and through the coupling with the breast pumping part 122, the cover part 121 accommodates the breast pump 130, the connector 140 and the controller 150 therein.

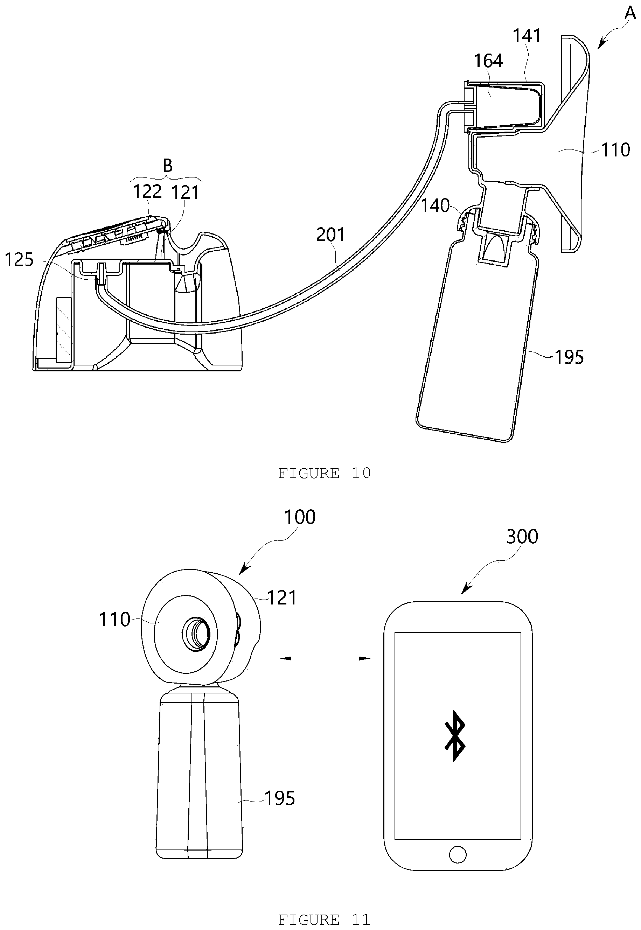

If the breast pumping part 122 is used as a separate breast pumping device, in addition, an air hose connection hole 125 is formed on the breast pumping part 122 to connect an air hose 201 (See FIG.10) thereto.

Further, the connector 140 is formed integrally with the backflow prevention part 141 at a coupled side to the air hose 201.

The backflow prevention part 141 serves to block the introduction of foreign matters if the breast pumping machine 100 is used as a separate type breast pumping machine, and the backflow prevention elastic member 160 made of a silicone material is insertedly disposed in the backflow prevention part 141. As shown in FIG.8, the smart wearable breast pumping machine 100 is coupled to the breast milk container 195 such as a feeding bottle, a breast milk storage pack, and the like and thus collects the breast milk.

Like this, the smart wearable breast pumping machine 100 according to the first embodiment of the present invention has an integral type structure in which the breast pump is built, thereby making it convenient to be carried and used.

Moreover, the smart wearable breast pumping machine 100 according to the first embodiment of the present invention has a function of nipple correction.

In more detail, the breast milk container 195 or stopper is coupled to the connector 140, and if the suction part 110 is attached to the user's breast, next, internal spaces of the suction part 110 and the connector 140 are sealed, so that if the breast pump 130 is operated, the user's nipple can be corrected by means of the breast pumping pressure of the breast pump 130.

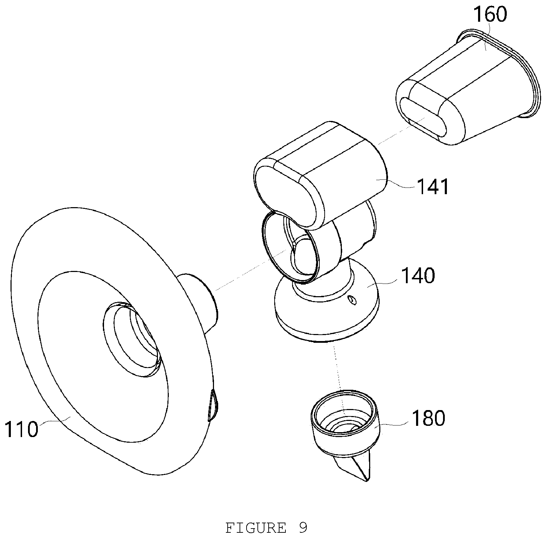

FIG.9 is an exploded perspective view showing the smart wearable breast pumping machine according to the first embodiment of the present invention in case of the smart wearable breast pumping machine with a separate type, and FIG.10 is a perspective view showing a method for using the separate type smart wearable breast pumping machine according to the first embodiment of the present invention.

As shown in FIG.9, if it is desired that the smart wearable breast pumping machine 100 according to the first embodiment of the present invention is used as a separate type breast pumping machine, the suction part 110 and the connector 140 are separated from the breast pumping part 122.

As a result, as shown in FIG.10, the breast milk container 195 is coupled to the connector 140, which serves as a breast milk collector A, and the breast pumping part 122 and the cover part 121 constitute a separate breast pumping device B.

Accordingly, the breast pumping pressure applied from the breast pumping device B is transferred to the breast milk collector A through the air hose 201, thereby performing the breast pumping.

In more detail, the breast pumping pressure applied from the breast pump 130 disposed in the breast pumping part 122 of the breast pumping device B is transferred to the suction part 110 through the connector 140 of the breast milk collector A through the air hose 201, thereby performing the breast pumping.

As a result, as shown in FIGS.9 and 10, the smart wearable breast pumping machine 100 according to the first embodiment of the present invention is separated into the breast milk collector A and the breast pumping device B, thereby providing a convenient breast pumping environment according to the user's need.

Moreover, the connector 140 is formed integrally with the backflow prevention part 141 at a coupled side to the air hose 201.

The backflow prevention part 141 serves to block introduction of foreign matters, and the backflow prevention elastic member 160, which is made of a silicone material, is insertedly disposed in the backflow prevention part 141.

Accordingly, the backflow prevention part 141 prevents the foreign matters from entering the breast milk collector A from the breast pumping device B by means of the pressure generated at the time when the breast pumping device B is operated.

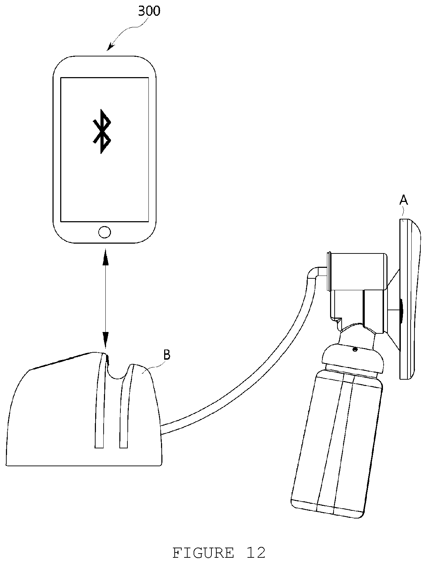

FIGS.11 and 12 show a communication method between the smart wearable breast pumping machine and a user terminal. In more detail, FIG.11 is a perspective view showing a communication method between the integral type breast pumping machine according to the first embodiment of the present invention and a user terminal, and FIG.12 is a perspective view showing a communication method between the separate type breast pumping machine according to the first embodiment of the present invention and a user terminal.

As shown in FIGS.11 and 12, the smart wearable breast pumping machine 100 according to the first embodiment of the present invention further includes a communication module adapted to transmit breast pumping information and operation information of the breast pump to a user terminal 300 and to receive the control commands from the user terminal 300.

In more detail, as shown in FIG.11, if the smart wearable breast pumping machine 100 is used as the integral type breast pumping machine, the breast pumping part 122 transmits the breast pumping information and the operation information of the breast pump to the user terminal 300 and receives the control commands from the user terminal 300. As shown in FIG.12, if the smart wearable breast pumping machine 100 is used as the separate type breast pumping machine, the breast pumping device B transmits the breast pumping information and the operation information of the breast pump to the user terminal 300 and receives the control commands from the user terminal 300.

Moreover, the communication module is a Bluetooth module performing Bluetooth communication for the communication with the user terminal 300 and for the convenience of use.

Further, the user terminal 300 installs and executes an application to receive the information from the smart wearable breast pumping machine 100 and to transmit the control commands to the smart wearable breast pumping machine 100. Accordingly, the user can check the state of the smart wearable breast pumping machine 100 and the breast pumping information very easily and intuitively through graphic elements displayed with images, graphs or numerical values on the application provided by the user terminal 300.

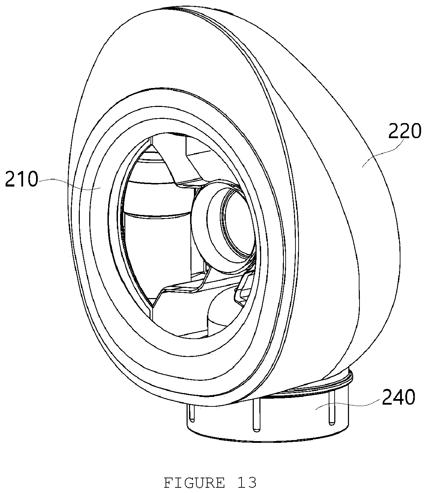

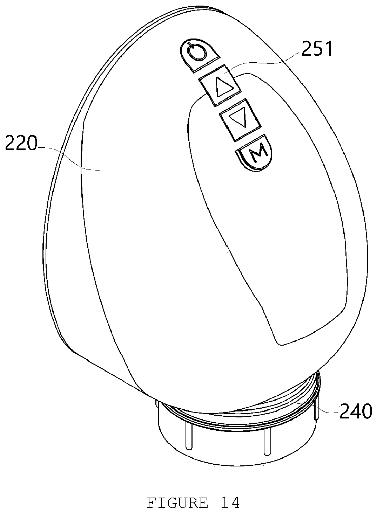

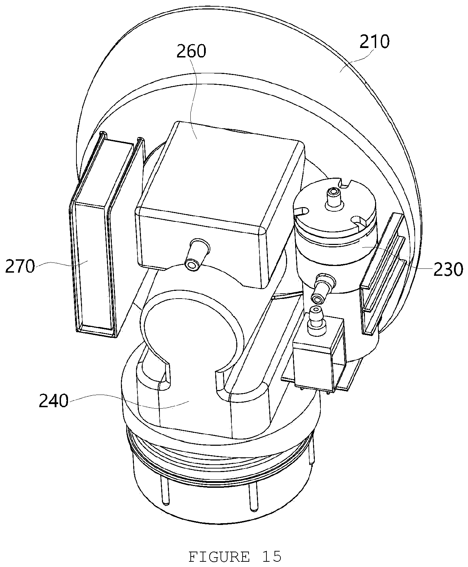

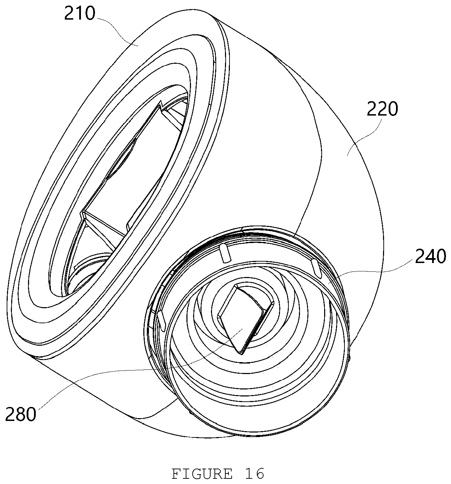

FIGS.13 and 14 are perspective views showing an outer appearance of a smart wearable breast pumping machine according to a second embodiment of the present invention, FIG.15 is a perspective view showing a bottom portion of the smart wearable breast pumping machine according to the second embodiment of the present invention, and FIG.16 is a perspective view showing an interior of the smart wearable breast pumping machine according to the second embodiment of the present invention.

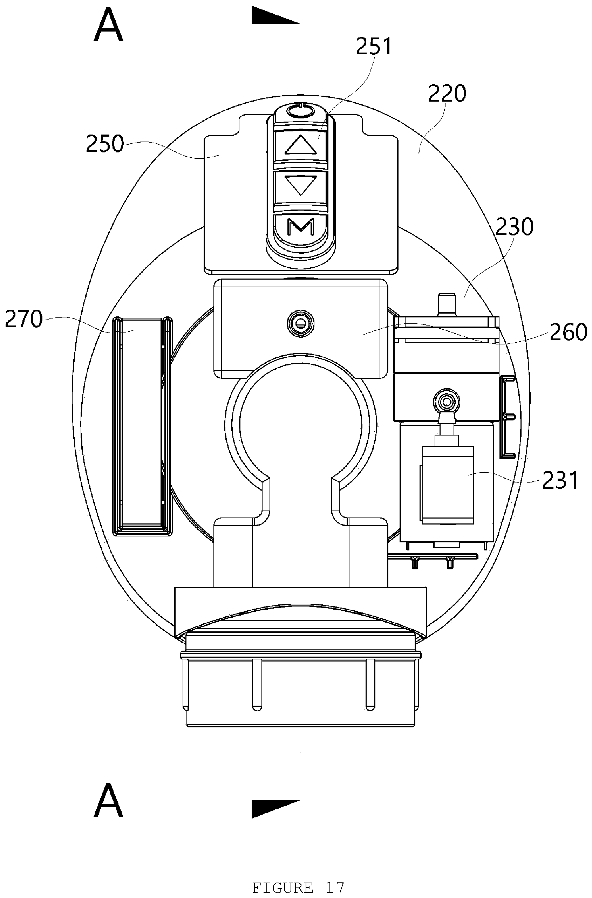

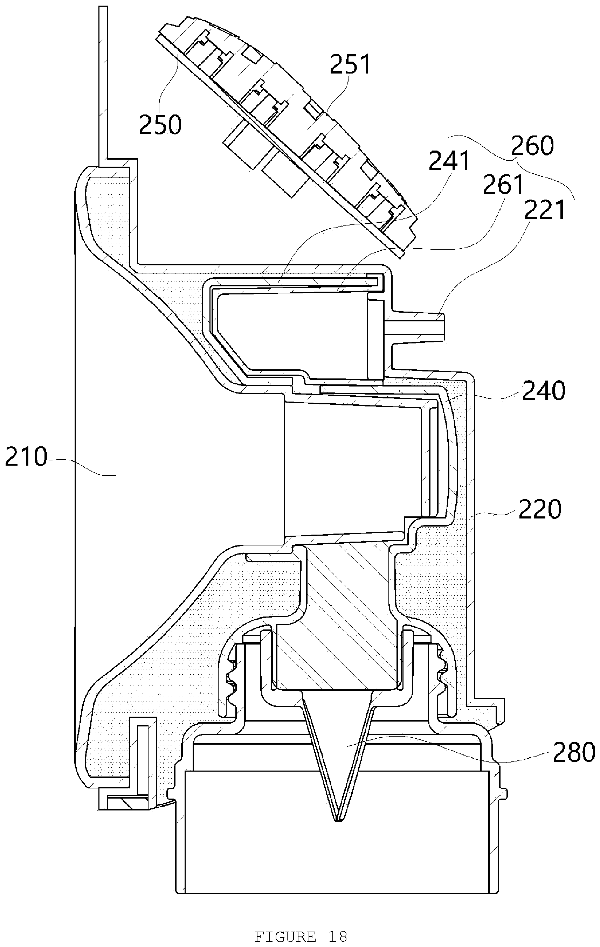

Further, FIG.17 is a front view showing the interior of the smart wearable breast pumping machine according to the second embodiment of the present invention, and FIG.18 is a sectional view showing the smart wearable breast pumping machine according to the second embodiment of the present invention, which is taken along the line A-A' of FIG.17.

Now, an explanation on the smart wearable breast pumping machine according to the second embodiment of the present invention will be given with reference to FIGS.13 to 18.

As shown in FIGS.13 to 18, the smart wearable breast pumping machine according to the second embodiment of the present invention includes a suction part 210, a cover part 220, a breast pump 230, a connector 240, a controller 250, and a backflow prevention part 260.

As shown in FIGS. 13 and 14, the suction part 210 and the cover part 220 are coupled to each other, and a button type command input part 251 is disposed on the cover part 220 to control operation, stop, and strength adjustment for the breast pumping machine.

The suction part 210 comes into close contact with a user's breast and is thus attached thereto. Further, the breast pump 230 applies a breast pumping pressure to the suction part 210, thereby pumping breast milk.

As shown in FIGS.15 and 16, the connector 240 has one side connected to the suction part 210 to introduce the breast milk pumped by means of the breast pumping pressure therein and the other side coupled to a breast milk container such as a feeding bottle, a breast milk storage pack, and the like.

Accordingly, the breast pumping pressure received from the breast pump 230 is applied to the suction part 210 attached to the user's breast, thereby allowing the breast milk to be pumped, and the pumped breast milk is introduced and stored in the breast milk container through the connector 240 and a silicone head 280.

The controller 250 controls the breast pump 230 through the control commands to allow the operation, stop, and strength adjustment for the breast pumping machine to be controlled, and the command input part 251 for inputting the control commands of the breast pumping machine is exposed to the outside through the cover part 220.

As shown in FIGS.17 and 18, the cover part 220 is coupled to the suction part 210 to form an outer appearance of the smart wearable breast pumping machine.

Through the coupling relation of the cover part 220 with the suction part 210, in more detail, the cover part 220 accommodates the breast pump 230, the connector 240, and the controller 250 therein, thereby forming the smart wearable breast pumping machine.

Further, the backflow prevention part 260 serves to prevent the foreign matters from flowing back from the breast pump 230. In more detail, the backflow prevention part 260 serves to block the foreign matters introduced from the interior of the breast pump 230 by means of the breast pumping pressure applied to the connector 240 at the time when the breast pump 230 is operated.

After a sucking force is generated from the breast pump 230 to pump the breast milk, in detail, the breast pump 230 temporarily releases the generated sucking force to generate next sucking force, and during this process, the foreign matters may be introduced through the air hose from the breast pump 230. Accordingly, the backflow prevention part 260 serves to block the introduction of the foreign matters.

So as to allow the smart wearable breast pumping machine to become compact in configuration, the backflow prevention part 260 is disposed on top of the connector 240, and so as to allow the foreign matters to be blocked effectively, the backflow prevention part 260, which is connected to the breast pump 230 by means of the air hose, further includes an elastic member 261 disposed therein in such a manner as to be elastically deformed. Accordingly, the elastic member 261 is elastically deformed according to the breast pumping pressure applied from the breast pump 230.

As shown in FIG.18, the backflow prevention part 260 includes a case 241, a cap 221, and the elastic member 261.

In more detail, the case 241 is extended from the connector 240 in such a manner as to be formed unitarily with the connector 240.

Further, the cap 221 is extended from the cover part 220 in such a manner as to be formed unitarily with the cover part 220. The cap 221 is coupled to the case 241 and is also coupled to the air hose connected to the breast pump 230.

The elastic member 261 is disposed in an interior formed by coupling the case 241 and the cap 221 to each other and is elastically deformed by means of the breast pumping pressure applied from the breast pump 230.

So as to allow the smart wearable breast pumping machine to be configured as a more compact integral type hands-free breast pumping machine, the internal parts of the smart wearable breast pumping machine may be formed integrally with the cover part 220 at the inside of the structure where the suction part 210 and the cover part 220 are coupled to each other.

In more detail, the cover part 220 has a compact structure in which an outer appearance of the breast pump 230, a controller housing for accommodating the controller 250, a battery housing for accommodating a battery 270 applying power to the breast pump 230, and an outer appearance of the backflow prevention part 260 are formed unitarily with the inside coupled to the suction part 210, thereby providing the smart wearable breast pumping machine capable of lowering a manufacturing cost and being conveniently carried and used.

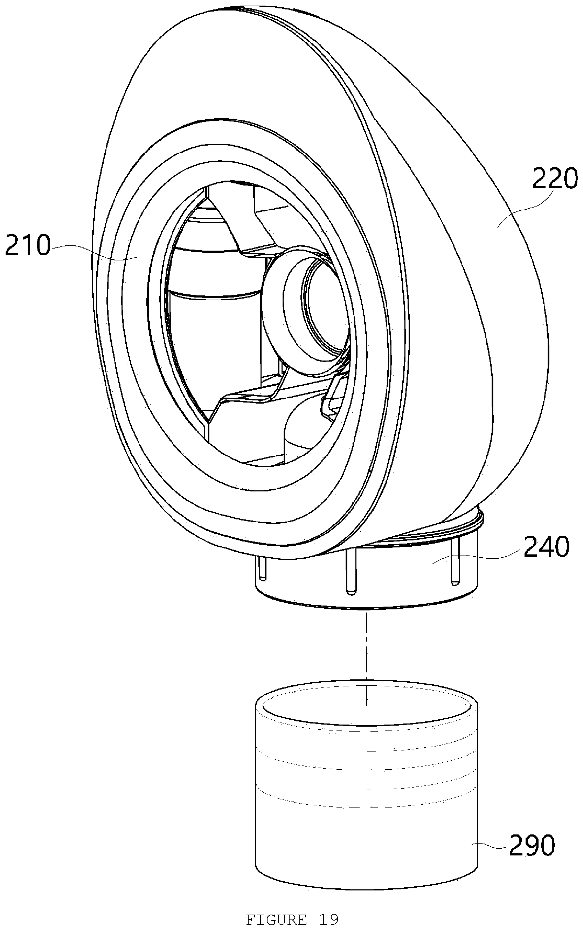

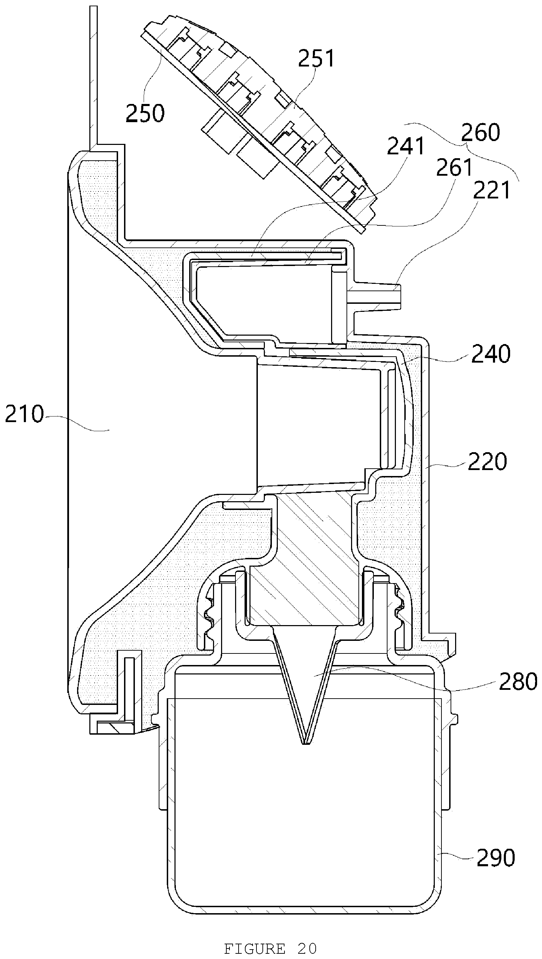

FIG.19 is a perspective view showing a smart wearable breast pumping machine according to a third embodiment not falling under the claimed invention, and FIG.20 is a sectional view showing the smart wearable breast pumping machine according to the third embodiment.

Now, an explanation on a smart wearable breast pumping machine according to a third embodiment will be given with reference to FIGS.19 and 20.

A smart wearable breast pumping machine according to a third embodiment has a function of nipple correction.

The smart wearable breast pumping machine according to the third embodiment includes a suction part 210, a cover part 220, a breast pump 230, a connector 240, a controller 250, and a backflow prevention part 260. Further, the smart wearable breast pumping machine according to the third embodiment includes a stopper 280.

The smart wearable breast pumping machine according to the third embodiment is configured to couple the stopper 290 to the connector 240.

If the suction part 210 is attached to the user's breast, accordingly, internal spaces of the suction part 210 and the connector 240 are sealed by means of the stopper 290, so that if the breast pump 230 is operated, the user's nipple can be corrected by means of the breast pumping pressure of the breast pump 230.

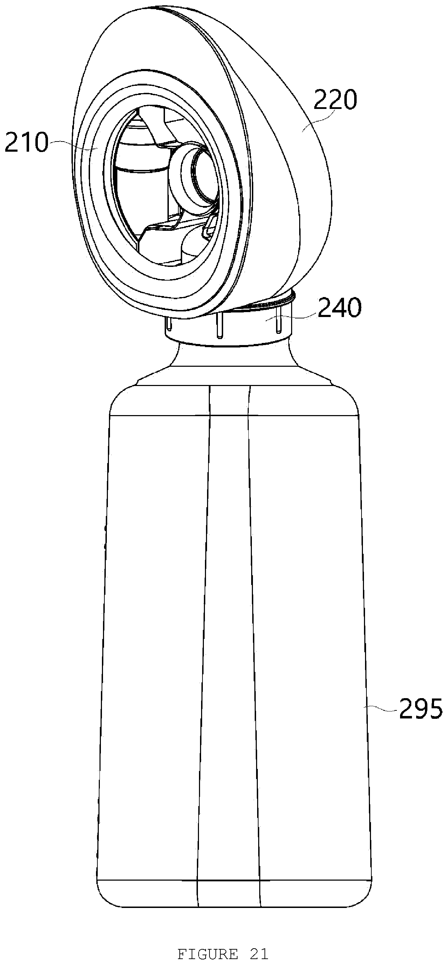

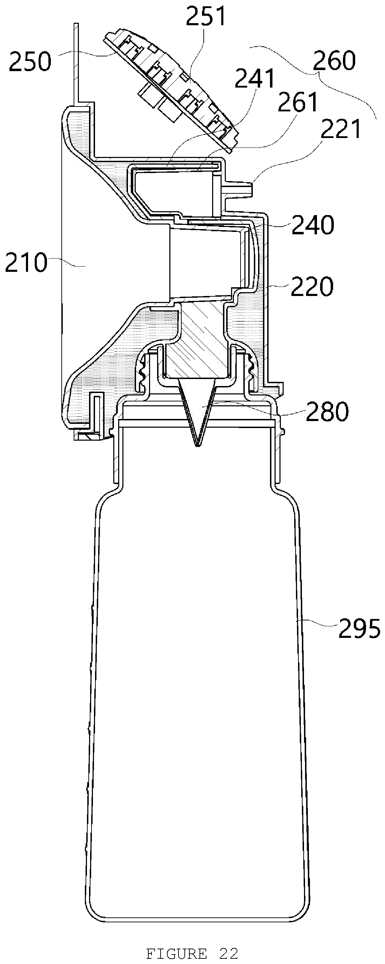

FIG.21 is a perspective view showing a smart wearable breast pumping machine according to a fourth embodiment of the present invention, and FIG.22 is a sectional view showing the smart wearable breast pumping machine according to the fourth embodiment of the present invention.

A smart wearable breast pumping machine according to a fourth embodiment of the present invention includes a suction part 210, a cover part 220, a breast pump 230, a connector 240, a controller 250, and a backflow prevention part 260. Further, the smart wearable breast pumping machine according to the fourth embodiment of the present invention includes a breast milk container 295.

The smart wearable breast pumping machine according to the fourth embodiment of the present invention is configured to couple the breast milk container 295 to the connector 240.

If the suction part 210 is attached to the user's breast, accordingly, internal spaces of the suction part 210 and the connector 240 are sealed by means of the breast milk container 295, so that if the breast pump 230 is operated, the user's nipple can be corrected by means of the breast pumping pressure of the breast pump 230.

As set forth in the foregoing, the smart wearable breast pumping machine according to the present invention has the integral type structure in which the breast pump is built and also has the separate type structure in which the breast pump is not built therein and is provided as the separate type breast pumping device, thereby making breast pumping environments corresponding to a variety of needs of the user.

In addition, the smart wearable breast pumping machine according to the present invention receives and controls the information therefrom through the application installed on the user terminal, thereby providing more accurate and useful breast pumping information for the user.

While the present invention has been described with reference to the particular illustrative embodiments, it is not to be restricted by the embodiments but only by the appended claims.

## Claims

1. A smart wearable breast pumping machine comprising:
a suction part (110) attached to a user's breast;
a breast pump (130), (230) for applying a breast pumping pressure to the suction part (110);
a connector (140) connected to the suction part (110) to introduce breast milk pumped by means of the breast pumping pressure and coupled to a breast milk container;
a controller (150)for controlling operations of the breast pump (130), (230) by means of control commands; and
a breast pumping part (122) coupled to the suction part(110) to accommodate the connector (140) the breast pump (130), (230) adapted to apply the breast pumping pressure to the suction part (110) and the controller (150) adapted to control operations of the breast pump by means of control commands,
the breast pumping part (122) further comprises a communication module adapted to transmit breast pumping information of the breast milk and operation information of the breast pump (130), (230) to a user terminal and to receive the control commands from the user terminal, a cover part (121) (220) coupled to the suction part (110)to accommodate the breast pump (130), (230), the connector (140) and the controller (150) therein,
**characterized in that** it
further comprises a backflow prevention part (141) disposed on the connector (140) to prevent foreign matters from flowing back by means of the breast pumping pressure applied to the connector (140)from the breast pump (130), (230, wherein the backflow prevention part (141) comprises:
a case extended from the connector (140) in such a manner as to be formed unitarily with the connector(140);
a cap extended from the cover part (121) (220) in such a manner as to be formed unitarily with the cover part (121) (220), coupled to the case, and coupled to an air hose (125) connected to the breast pump (130), (230);
an elastic member (160), (261) disposed in an interior formed by coupling the case and the cap to each other in such a manner as to be elastically deformed by means of the breast pumping pressure applied from the breast pump (130), (230) through the air hose.

2. The smart wearable breast pumping machine according to claim 1, wherein the breast pumping part (122) is separated from the suction part (110) and the connector (140)to constitute a separate breast pumping device.

3. The smart wearable breast pumping machine according to claim 2, wherein the suction part (110) and the connector(140) are separated from the breast pumping part (122) to constitute a separate breast milk collector, and the connector (140)receiving the breast pumping pressure from the breast pumping part (122)through an air hose transfers the breast pumping pressure to the suction part (110) to allow the pumped breast milk to be collected to the breast milk collector.

4. The smart wearable breast pumping machine according to claim 1, wherein the cover part (121) (220)is formed unitarily with a breast pump outer appearance portion for forming an outer appearance of the breast pump, a controller (150) housing for accommodating the controller (150), and a battery housing for accommodating a battery applying power to the breast pump, on the inside coupled to the suction part (110) .

5. The smart wearable breast pumping machine according to claim 1, further comprising a stopper or breast milk container coupled to the connector (140).

## Patentansprüche

1. Intelligente, tragbare Brustpumpenmaschine, umfassend:
ein Saugteil (110), das an der Brust eines Benutzers angebracht wird;
eine Brustpumpe (130), (230) zum Anlegen eines Brustpumpdrucks an das Saugteil (110);
ein Verbindungsstück (140), das mit dem Saugteil (110) zum Einleiten der mittels des Brustpumpdrucks abgepumpten Muttermilch verbunden und an einen Muttermilchbehälter gekoppelt ist;
eine Steuerung (150) zum Steuern des Betriebs der Milchpumpe (130), (230) mittels Steuerbefehlen; und
ein Brustpumpenteil (122), das mit dem Saugteil (110) gekoppelt ist, um das Verbindungsstück (140) aufzunehmen, wobei die Brustpumpe (130), (230) dazu geeignet ist, den Brustpumpdruck auf das Saugteil (110) auszuüben, und die Steuerung (150) dazu geeignet ist, den Betrieb der Brustpumpe mittels Steuerbefehlen zu steuern,
wobei der Brustpumpenteil (122) ferner ein Kommunikationsmodul umfasst, das dazu geeignet ist, Brustpumpinformationen der Muttermilch und Betriebsinformationen der Brustpumpe (130), (230) an ein Benutzerterminal zu übertragen und die Steuerbefehle von dem Benutzerterminal zu empfangen,
ein Abdeckteil (121) (220), das mit dem Saugteil (110) gekoppelt ist, um die Brustpumpe (130), (230), das Verbindungsstück (140) und die Steuerung (150) darin unterzubringen,
**dadurch gekennzeichnet, dass** sie ferner ein Rückflussverhinderungsteil (141) umfasst, das an dem Verbindungsstück (140) angeordnet ist, um zu verhindern, dass Fremdkörper mittels des Brustpumpdrucks, der von der Brustpumpe (130), (230) auf das Verbindungsstück (140) ausgeübt wird, zurückfließen, wobei
der Rückflussverhinderungsteil (141) Folgendes umfasst:
ein Gehäuse, das sich von dem Verbindungsstück (140) so erstreckt, dass es als Einheit mit dem Verbindungsstück (140) bildet gebildet wird;
eine Kappe, die sich von dem Abdeckteil (121) (220) so erstreckt, dass sie mit dem Abdeckteil (121) (220) eine Einheit bildet, mit dem Gehäuse gekoppelt ist und mit einem Luftschlauch (125) gekoppelt ist, der mit der Milchpumpe (130), (230) verbunden ist;
ein elastisches Element (160), (261), das in einem Innenraum angeordnet ist, der durch Koppeln des Gehäuses und der Kappe miteinander gebildet wird, sodass es mittels des von der Milchpumpe (130), (230) durch den Luftschlauch ausgeübten Brustpumpdrucks elastisch verformt wird.

2. Intelligente, tragbare Brustpumpmaschine nach Anspruch 1, wobei der Brustpumpteil (122) von dem Saugteil (110) und dem Verbindungsstück (140) getrennt ist, um eine separate Brustpumpvorrichtung auszubilden.

3. Intelligente, tragbare Brustabpumpmaschine nach Anspruch 2, wobei der Saugteil (110) und das Verbindungsstück (140) von dem Brustpumpteil (122) getrennt sind, um einen separaten Brustmilchsammler auszubilden, und das Verbindungsstück (140), das den Brustpumpdruck von dem Brustpumpteil (122) über einen Luftschlauch aufnimmt, den Brustpumpdruck zu dem Saugteil (110) überträgt, damit die abgepumpte Muttermilch in dem Brustmilchsammler gesammelt werden kann.

4. Intelligente, tragbare Brustpumpmaschine nach Anspruch 1, wobei das Abdeckteil (121) (220) als Einheit mit einem äußeren Erscheinungsbildabschnitt der Brustpumpe zum Bilden eines äußeren Erscheinungsbildes der Brustpumpe, einem Gehäuse für die Steuerung (150) zum Aufnehmen der Steuerung (150) und einem Batteriegehäuse zum Aufnehmen einer Batterie, die die Brustpumpe mit Strom versorgt, auf der Innenseite, die mit dem Ansaugteil (110) gekoppelt ist, gebildet ist.

5. Intelligente, tragbare Brustabpumpmaschine nach Anspruch 1, die ferner einen Stopfen oder einen Muttermilchbehälter umfasst, der mit dem Verbindungsstück (140) gekoppelt ist.

## Revendications

1. Machine à tirer le lait à portable et intelligente comprenant :
une partie d'aspiration (110) fixée au sein d'une utilisatrice ;
un tire-lait (130), (230) pour appliquer une pression de tire-lait à la partie d'aspiration (110) ;
un raccord (140) raccordé à la partie d'aspiration (110) pour introduire le lait maternel pompé au moyen de la pression de tire-lait et accouplé à un récipient pour lait maternel ;
un dispositif de commande (150) pour commander les opérations du tire-lait (130), (230) au moyen d'instructions de commande ; et
une partie tire-lait (122) accouplée à la partie d'aspiration (110) pour recevoir le raccord (140) du tire-lait (130), (230) adapté à appliquer la pression de tire-lait à la partie d'aspiration (110) et le dispositif de commande (150) adapté à commander les opérations du tire-lait au moyen d'instructions de commande,
la partie tire-lait (122) comprend en outre un module de communication adapté à transmettre des informations de tire-lait du lait maternel et des informations d'opération du tire-lait (130), (230) à un terminal utilisateur et pour recevoir les instructions de commande du terminal utilisateur,
une partie couvercle (121) (220) accouplée à la partie d'aspiration (110) pour recevoir le tire-lait (130), (230), le raccord (140) et le dispositif de commande (150) à l'intérieur,
**caractérisée en ce qu'**elle comprend en outre une partie anti-reflux (141) disposée sur le raccord (140) pour empêcher les corps étrangers de refluer au moyen de la pression de tire-lait appliquée au raccord (140) depuis le tire-lait (130), (230, dans laquelle
la partie anti-reflux (141) comprend :
un boîtier s'étendant à partir du raccord (140) de manière à être formé d'une seule pièce avec le raccord (140) ;
un capuchon s'étendant depuis la partie couvercle (121) (220) de manière à être formé d'un seul tenant avec la partie couvercle (121) (220), accouplé au boîtier, et accouplé à un tuyau d'air (125) raccordé au tire-lait (130), (230) ;
un élément élastique (160), (261) disposé dans un intérieur formé en accouplant le boîtier et le capuchon l'un à l'autre de manière à être déformé élastiquement au moyen de la pression de tire-lait appliquée depuis le tire-lait (130), (230) à travers le tuyau d'air.

2. Machine à tirer le lait portable et intelligente selon la revendication 1, dans laquelle la partie tire-lait (122) est séparée de la partie d'aspiration (110) et du raccord (140) pour constituer un dispositif de tire-lait séparé.

3. Machine à tirer le lait portable et intelligente selon la revendication 2, dans laquelle la partie d'aspiration (110) et le raccord (140) sont séparés de la partie tire-lait (122) pour constituer un collecteur de lait maternel séparé, et le raccord (140) recevant la pression de tire-lait provenant de la partie tire-lait (122) à travers un tuyau d'air transfère la pression de tire-lait à la partie d'aspiration (110) pour permettre au lait maternel pompé d'être collecté vers le collecteur de lait maternel.

4. Machine à tirer le lait portable et intelligente selon la revendication 1, dans laquelle la partie couvercle (121) (220) est formée d'un seul tenant avec une partie d'apparence externe de tire-lait pour former une apparence externe du tire-lait, un boîtier de dispositif de commande (150) pour recevoir le dispositif de commande (150), et un boîtier de batterie pour recevoir une batterie appliquant de l'énergie au tire-lait, sur l'intérieur accouplé à la partie d'aspiration (110).

5. Machine à tirer le lait portable et intelligente selon la revendication 1, comprenant en outre un bouchon ou un récipient pour lait maternel accouplé au raccord (140).
